Europäisches Patentamt

**European Patent Office**

Office européen des brevets

(11) Publication number : **0 449 810 B1**

# EUROPEAN PATENT SPECIFICATION

(45) Date of publication of patent specification :
**31.03.93 Bulletin 93/13**

(51) Int. Cl.⁵ : **A61K 33/06, A61K 33/14**

(21) Application number : **89901098.7**

(22) Date of filing : **23.12.88**

(86) International application number :
**PCT/DK88/00220**

(87) International publication number :
**WO 90/07338 12.07.90 Gazette 90/16**

(54) **CALCIUM CHLORIDE CONTAINING PREPARATION FOR THE PREVENTION OR THE TREATMENT OF HYPOCALCEMIA IN RUMINANTS.**

(43) Date of publication of application :
**09.10.91 Bulletin 91/41**

(45) Publication of the grant of the patent :
**31.03.93 Bulletin 93/13**

(84) Designated Contracting States :
**AT BE CH DE FR GB IT LI NL SE**

(56) References cited :
**DE-C- 365 554
US-A- 3 996 351
US-A- 4 346 077
Chemical absracts, Vol. 34 (1940), abstract No. 3875, Rev. farm. (Buenos Aires) 81, 15-22, 40-54 (1939)
Chemical abstracts, Vol. 57(1962), abstract No. 5560a, Izv. Estestv.-Nauchn Inst. pri Permsk. Univ. 14, No. 4, 67-76 (1960)**

(56) References cited :
**Chemical Abstracts, Vol. 104 (1986), abstract No. 219148d, Jpn. Kokai Tokkyo Koho JP 61 36, 222 (86 36, 222)
"Remington's Pharmaceutical Sciences", 15 Ed., published 1975, by Mack Publishing Company (Easton, Pennsylvania), see page 1258 (Calcium Sulfate NF)**

(73) Proprietor : **BOEHRINGER INGELHEIM AGROVET A/S
Rygaards Alle 131
DK-2900 Hellerup (DK)**

(72) Inventor : **NIELSEN, Leif, Hojvang
Stefansgade 3
D-2200 Kobenhavn N (DK)**

(74) Representative : **Kyed, Iver et al
c/o LEHMANN & REE A/S Grundtvigsvej 37
DK-1864 Frederiksberg C (DK)**

EP 0 449 810 B1

Jouve, 18, rue Saint-Denis, 75001 PARIS

## Description

The present invention relates to a calcium containing preparation for the prevention or the treatment of hypocalcemia in ruminants.

During the period pending calving when the cow's milk production has normally stopped completely its calcium mobilisation has also stopped.

Calving sets off a high milk production and consequently a considerable need for calcium arises. In order to compensate for this sudden need the body tries to provide calcium from the blood thus causing the calcium ion content of the blood to decrease drastically. This causes the cow to lose control of its muscles, to lie down and become increasingly apathetic. Unless treated the cow goes into a coma and dies. The condition thus occurred is referred to as milk fever. Older cows are particularly prone to milk fever.

It is known to treat milk fever by oral administration to the sick animal of an aqueous calcium chloride solution. The dosage is typically 400 ml solution corresponding to a total amount of about 500 g and this dosage is typically administered at 12 hours' intervals. Aqueous calcium chloride solutions are very unpleasantly tasting and therefore the sick animal must be force-treated. This may cause the calcium chloride solution to enter the lungs which may be fatal to the animal.

In normal practise the calcium chloride solution is replaced with a calcium chloride containing gel to prevent the therapeutic preparation from entering the lungs. This course of action is only followed for lack of a better one since the use of a gel poses dosage problems due to the difficulties associated with the discharge of the gel from the container in which it is packed. The use of a gel does not eliminate the taste problem either.

Attempts have also been made to treat animals suffering from milk fever with capsules containing a powdery calcium compound, such as calcium acetochloride. However, the calcium content of such capsules is relatively low and consequently a considerable number of capsules (e.g. 16) should be administered to the sick animal at two hours' intervals thus rendering the preparation unsuitable for practical use.

Surprisingly it has now been found that a solid and easily doseable preparation having a high calcium content may be produced from an aqueous mixture of calcium chloride ($CaCl_2$) and calcium sulphate ($CaSO_4$).

Thus the preparation according to the invention is characterized in that it has been produced by solidification of a mixture of $CaCl_2,xH_2O$ and $CaSO_4,yH_2O$, wherein x is a number equal to or higher than 0 but lower than or equal to 6 and y is a number equal to or higher than 0 but lower than or equal to 2, and water, the weight ratio of $CaCl_2,xH_2O$ to $CaSO_4,yH_2O$ being from 1:0.05 to 1:2.4 and preferably from 1:0.11 to 1:2.4 and the water content being sufficiently high for making the mixture pumpable but constituting no more than the amount required to convert any $CaCl_2,xH_2O$ present into $CaCl_2,6H_2O$ and $CaSO_4,yH_2O$ into $CaSO_4,2H_2O$.

The term "pumpable" denotes that at a temperature of 40°C the mixture is capable of flowing through a funnel having a neck-diameter of 10 mm.

Immediately upon manufacture a mixture as described above has a typical viscosity like that of double cream. In this state the mixture may readily be enclosed in capsules, e.g. gelatine capsules. After a while, typically 1-2 hours, the mixture solidifies and a solid substance is produced.

The use of the above mixture for the production of calcium containing gelatine capsules provides the particular advantage that the gelatine is not attacked by the mixture and that a substantially larger amount of Ca (in the form of $Ca^{++}$) may be added to the capsules as compared to the known capsules described above.

The production of the above mixture is preferably carried out by admixing the calcium chloride and the calcium sulphate in their dry state to form a homogeneous mixture. Water is then added and mixing is continued until a pumpable mass is produced. This mass is preferably encapsulated in gelatine capsules in an amount of e.g. 80-100 g per capsule. The filled gelatine capsules are allowed to stand at a suitable temperature, e.g. at room temperature, for a sufficient period for the mixture to solidify. The solidification is a result of the bonding of the water in the form of crystal water.

Instead of being encapsulated in gelatine capsules the mixture may be cast to form suitable dosage units. It may also be cast in the form of a coherent mass which may then be disintegrated and ground to form a powder for therapeutic use or for pelleting.

The preparation according to the invention is particularly suitable for the treatment of milk fever but it is also suitable for the treatment of hypocalcemia in other ruminants, e.g. sheep.

The invention will now be described in further detail with reference to the following example.

### Example

Calcium containing gelatine capsules were produced as follows from solid $CaCl_2,2H_2O$ and $CaSO_4,\frac{1}{2}H_2O$:

The solids were mixed in the amounts given in Table 1 below in a Björn mixer. Water was added to the mixture and mixing was continued for another 10-20 minutes until the mixture was homogeneous and easy-flowing. The mass thus obtained was then enclosed in gelatine capsules and the filled capsules were allowed to stand for 24 hours at room temperature in an airtight compartment.

The capsules were then packed in an airtight package.

### Table 1

| Test | $CaCl_2, 2H_2O$ g | $CaSO_4, \frac{1}{2}H_2O$ g | $H_2O$ g | $CaCl_2 \cdot 2H_2O : CaSO_4 \cdot \frac{1}{2}H_2O$ Mixture ratio |
|---|---|---|---|---|
| a: | 1000 | 0 | 520 | 1:0 |
| b: | 900 | 100 | 180 | 1:0.11 |
| c: | 800 | 200 | 170 | 1:0.25 |
| d: | 700 | 300 | 150 | 1:0.43 |
| e: | 600 | 400 | 150 | 1:0.67 |
| f: | 500 | 500 | 150 | 1:1.00 |
| g: | 400 | 600 | 170 | 1:1.50 |
| h: | 300 | 700 | 220 | 1:2.33 |
| i: | 200 | 800 | 350 | 1:4.00 |
| j: | 100 | 900 | 630 | 1:9.00 |
| k: | 0 | 1000 | 1200 | 1:$\infty$ |

Testing of the capsules produced yielded the results given in Table 2.

### Table 2

| Test | Solidification time, h | "Pumpability" | Compatibility with gelatine capsule | $Ca^{++}$ in filled capsule (g) |
|---|---|---|---|---|
| a: | partial solidification, 1/2 | poor | incompatible | 14.6 |
| b: | 1/2 | moderate | moderate | 19.6 |
| c: | 1-2 | good | " | 20.1 |
| d: | 3-6 | " | good | 20.6 |
| e: | 6-12 | " | " | 21.1 |
| f: | 12-18 | " | " | 21.4 |
| g: | 12-18 | " | " | 21.2 |
| h: | 18-24 | " | moderate | 20.6 |
| i: | >24 | " | incompatible | 19.0 |
| j: | >24 | " | " | 16.1 |
| k: | >24 | " | " | 12.3 |

As will appear from Table 2 only tests b-h employing the preparations according to the invention yields satisfactory results as regards both solidification time, compatibility with the gelatine capsule and the Ca content of the capsules.

The above results show the surprising synergistic effect of the simultaneous use of calcium chloride and calcium sulphate in admixture with water both as regards the compatibility of the mixture with gelatine capsules, pumpability and solidification time.

The surprising effect may be illustrated by comparative tests using (1) a mixture of 50 parts by weight of $CaCl_2,2H_2O$, 50 parts by weight of $CaSO_4,\frac{1}{2}H_2O$ and 15 parts by weight of water, (2) a mixture of 50 parts by weight of $CaCl_2,2H_2O$ and 15 parts by weight of water and (3) a mixture of 50 parts by weight of $CaSO_4,\frac{1}{2}H_2O$ and 15 parts by weight of water.

Whereas mixture (1) provides the desired compatibility with gelatine capsules and a satisfactory pumpability and solidification time mixture (2) forms a paste of crystals which is unpumpable and solidifies too quickly and which is also incompatible with gelatine capsules, and mixture (3) forms a mass with is neither pumpable nor castable.

## Claims

1. A calcium chloride containing preparation for the prevention or the treatment of hypocalcemia in ruminants and, characterized in that it has been produced by solidification of a mixture of $CaCl_2,xH_2O$ and $CaSO_4,yH_2O$, wherein x is a number equal to or higher than 0 but lower than or equal to 6 and y is a number equal to or higher than 0 but lower than or equal to 2, and water, the weight ratio of $CaCl_2,xH_2O$ to $CaSO_4,yH_2O$ being from 1:0.05 to 1:2.4 and the water content being sufficiently high for making the mixture pumpable but constituting no more than the amount required to convert any $CaCl_2,xH_2O$ present into $CaCl_2,6H_2O$ and $CaSO_4,yH_2O$ into $CaSO_4,2H_2O$.

2. A preparation according to claim 1, characterized in that it has been produced by solidification of a mixture of $CaCl_2,2H_2O$, $CaSO_4,\frac{1}{2}H_2O$ and water.

3. A preparation according to claim 1 or 2, characterized in that the weight ratio of $CaCl_2,xH_2O$ to $CaSO_4,yH_2O$ is from 1:0.11 to 1:2.4.

## Patentansprüche

1. Kalziumchlorid enthaltende Zubereitung zur Vorbeugung oder Behandlung von Hypocalcämie bei Wiederkäuern, dadurch gekennzeichnet, daß sie durch Verfestigen einer Mischung von $CaCl_2.xH_2O$ and $CaSO_4.yH_2O$, worin x eine Zahl gleich oder höher 0, jedoch niedriger als oder gleich 6 ist und y eine Zahl gleich oder höher als 0, jedoch niedriger als oder gleich 2 ist, und Wasser hergestellt worden ist, wobei das Gewichtsverhältnis von $CaCl_2.xH_2O$ zu $CaSO_4.yH_2O$ von 1 : 0,05 bis 1 : 2,4 beträgt und der Wassergehalt ausreichend hoch ist, um die Mischung pumpbar zu machen, jedoch nicht mehr ausmacht als die erforderliche Menge zur Umwandlung von beliebigem vorliegendem $CaCl_2.xH_2O$ in $CaCl_2.6H_2O$ und $CaSO_4.yH_2O$ in $CaSO_4.2H_2O$.

2. Zubereitung nach Anspruch 1, dadurch gekennzeichnet, daß sie durch Verfestigen einer Mischung von $CaCl_2.2H_2O$, $CaSO_4.1/2H_2O$ und Wasser hergestellt worden ist.

3. Zubereitung nach Anspruch 1 oder 2, dadurch gekennzeichnet, daß das Gewichtsverhältnis von $CaCl_2.xH_2O$ zu $CaSO_4.yH_2O$ von 1 : 0,11 bis 1 : 2,4 beträgt.

## Revendications

1. Préparation contenant du chlorure de calcium de prévention ou de traitement de l'hypocalcemie chez les ruminants et, **charactérisée en ce qu**'elle a été preparée par la solidification d'une mixture de $CaCl_2,xH_2O$ et $CaSO_4, yH_2O$, où x est un nombre égal à ou plus haut que 0 main plus bas que ou égal à 6, où y est un nombre égal à ou plus haut que 0 mais plus bas que ou égal à 2, et de l'eau, le ratio en poids entre $CaCl_2,xH_2O$ et $CaSO_4,yH_2O$ étant de 1:0,05 à 1:2,4 et le contenu en eau étant assez élevé pour que la mixture soit pompable en ne pas constituant plus que la quantité éxigée pour la conversion du $CaCl_2,xH_2O$ éventuellement présent en $CaCl_2,6H_2O$ et $CaSO_4,yH_2O$ en $CaSO_4,2H_2O$.

2. Préparation selon la revendication 1, **characterisée en ce qu**'elle a été preparée par la solidification d'une mixture de $CaCl_2,2H_2O$, $CaSO_4,\frac{1}{2}H_2O$ et d'eau.

3. Préparation selon la revendication 1 ou 2, **characterisée en ce que** le ratio en poids entre $CaCl_2,xH_2O$ et $CaSO_4,yH_2O$ est compris entre 1:0,11 et 1:2,4.